(19) Europäisches Patentamt / European Patent Office / Office européen des brevets

(11) **EP 4 046 570 A1**

(12) **EUROPEAN PATENT APPLICATION**

(43) Date of publication:
**24.08.2022   Bulletin 2022/34**

(21) Application number: **21202119.0**

(22) Date of filing: **12.10.2021**

(51) International Patent Classification (IPC):
**A61B 5/11** (2006.01)          **A61B 5/00** (2006.01)

(52) Cooperative Patent Classification (CPC):
**A61B 5/112; A61B 5/1122; A61B 5/4082;
A61B 5/4842; A61B 5/6823; A61B 5/6829;
A61B 5/7246; A61B 5/7264; A61B 5/7282;
A61B 5/742**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.01.2021   JP 2021002381**

(71) Applicant: **FUJITSU LIMITED
Kawasaki-shi, Kanagawa 211-8588 (JP)**

(72) Inventors:
• **Kusumine, Naruhiro
  Kanagawa, 211-8588 (JP)**
• **Hotta, Shinji
  Kanagawa, 211-8588 (JP)**
• **Inomata, Akihiro
  Kanagawa, 211-8588 (JP)**
• **Otsuka, Hiroshi
  Kanagawa, 211-8588 (JP)**

(74) Representative: **Hoffmann Eitle
Patent- und Rechtsanwälte PartmbB
Arabellastraße 30
81925 München (DE)**

(54) **GAIT STATE DETERMINATION SYSTEM, GAIT STATE DETERMINATION METHOD, AND GAIT STATE DETERMINATION PROGRAM**

(57)   A gait state determination system includes a sensor (50) attached to a body of a person, and an information processing device (10) configured to: acquire, using the sensor, data of a movement of at least a part of the body during a first period when the person is walking, identify a first feature amount of a first movement from the data acquired, and identify a second feature amount of a second movement from the data acquired, the first movement being determined in advance and corresponding to a symptom of the person, the second movement correlating with the first movement, make a determination regarding a gait state of the person based on the first feature amount of the first movement and the second feature amount of the second movement, and output a result of the determination.

FIG. 16

GAIT STATE OF PATIENT A

<MEASUREMENT RESULTS OF ALL BODY PARTS>

<MEASUREMENT RESULT OF COMPENSATION CHANGE>

THROUGH THE MEASUREMENT OF THE GAIT STATE OF THE PATIENT A, TWO MOVEMENTS THAT ARE TYPICAL MOVEMENTS OF PARKINSON'S DISEASE SYMPTOMS ARE OBSERVED. THE MOVEMENT APPEARING WHEN THE DISEASE IS SEVERE IS ALSO OBSERVED, AND THE SEVERITY IS EXPECTED TO BE 40%.

EP 4 046 570 A1

**Description**

FIELD

A certain aspect of embodiments described herein relates to a gait state determination system, a gait state determination method, and a gait state determination program.

BACKGROUND

Patients who develop muscular dystrophy, develop Parkinson's disease, or had a stroke may experience gait disorder. In the medical setting, the change in symptoms of the patient may be assessed by analyzing the gait state of the patient experiencing gait disorder. Conventionally, doctors or physical therapists visually check the gait state of the patient and estimate the progression of symptoms.

For example, Japanese Patent Application Publication No. 2012-179114 (Patent Document 1) discloses an art that measures the length of time for which both legs are in contact with the ground based on the output from sensors that measure the acceleration and the pressure applied to the human body to determine the deterioration in the locomotor function based on the ratio of the length of time for which both legs are in contact with the ground to the gait cycle.

SUMMARY

The results of assessment of the gait state of the patient obtained by using the art described in Patent Document 1 are not necessarily useful information in the medical setting. This is because, for example, patients with diseases that cause muscles to stiffen (e.g., muscular dystrophy) may be able to walk fast even if their diseases are very severe. On the other hand, to assist the insufficiency of the aimed motion, such patients may perform a movement (a compensating movement) in which another part of the body achieves the aimed motion instead of the diseased part. For example, patients whose muscle of the leg stiffens have difficulty in a typical gait movement. Thus, to walk forward, they perform the compensating movement in which they bend their upper bodies to lift the waists and put their legs forward. The compensating movements performed by patients differ depending on the types of diseases. Thus, to appropriately determine the gait disorder of the patient, the analysis needs to be tailored to symptoms of the patient.

The present invention is made in view of those circumstances, and an objective thereof is to provide a gait state determination system, a gait state determination method, and a gait state determination program that can accurately make a determination regarding the gait state of a person in accordance with symptoms.

According to an aspect of the embodiments, there is provided a gait state determination system including: a sensor attached to a body of a person; and an information processing device configured to: acquire, using the sensor, data of a movement of at least a part of the body during a first period when the person is walking, identify a first feature amount of a first movement from the data acquired, and identify a second feature amount of a second movement from the data acquired, the first movement being determined in advance and corresponding to a symptom of the person, the second movement correlating with the first movement, make a determination regarding a gait state of the person based on the first feature amount of the first movement and the second feature amount of the second movement, and output a result of the determination.

BRIEF DESCRIPTION OF DRAWINGS

FIG. 1 is a block diagram of an information processing system in accordance with an embodiment.
FIG. 2A illustrates types of data acquired from angular velocity sensors of sensing devices attached to legs and a waist, and FIG. 2B illustrates types of data acquired from acceleration sensors of the sensing devices attached to the legs and the waist.
FIG. 3 illustrates time-series data of horizontal rotation (waistGyroZ) acquired from the angular velocity sensor attached to the waist, and time series-data of the horizontal rotation acquired from each of the angular velocity sensors attached to ankles.
FIG. 4A illustrates hardware configurations of a physical therapist terminal and a doctor terminal, and FIG. 4B illustrates a hardware configuration of a server.
FIG. 5 is a functional block diagram of the server.
FIG. 6 illustrates a sensor data DB.
FIG. 7A is an enlarged view of an area H in FIG. 3, FIG. 7B illustrates a list of gait events (feature points), and FIG. 7C illustrates a list of analysis target sections.

FIG. 8 illustrates an analysis data DB.

FIG. 9A illustrates types of sensor data, and FIG. 9B illustrates types of analysis target sections.

FIG. 10 illustrates types and equations of statistics values.

FIG. 11A and FIG. 11B illustrate a pattern DB.

FIG. 12 illustrates a symptom DB.

FIG. 13 is a flowchart illustrating a pre-process executed by the sensing device.

FIG. 14 is a flowchart illustrating a data analysis process executed by the server.

FIG. 15 illustrates distribution of compensation changes of healthy people and patients with diseases.

FIG. 16 illustrates an output screen.

FIG. 17 illustrates the pattern DB in accordance with a variation of the embodiment.

FIG. 18 illustrates distribution of compensation changes of healthy people and patients with diseases.

## DESCRIPTION OF EMBODIMENTS

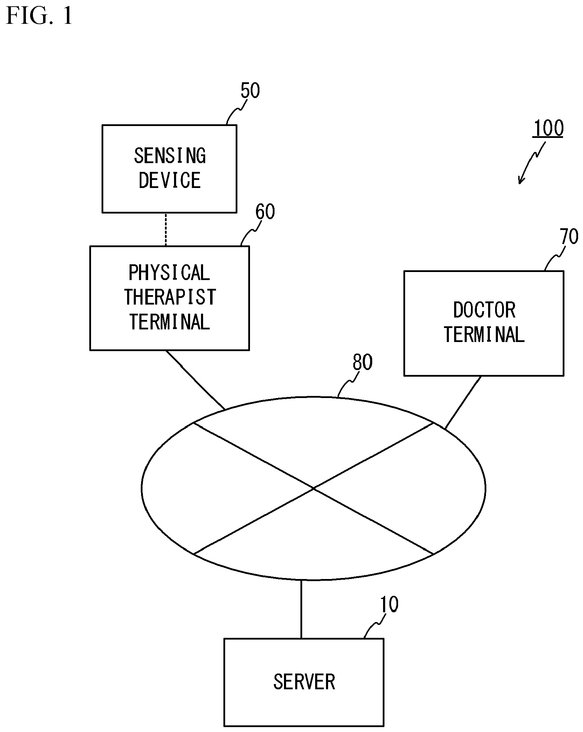

Hereinafter, an embodiment of an information processing system will be described with reference to FIG. 1 to FIG. 16. FIG. 1 schematically illustrates a configuration of an information processing system 100 in accordance with an embodiment.

As illustrated in FIG. 1, the information processing system 100 includes a sensing device 50, a physical therapist terminal 60, a doctor terminal 70, and a server 10 as an information processing device. The server 10, the physical therapist terminal 60, and the doctor terminal 70 are connected to a network 80 such as the Internet.

The sensing device 50 includes sensors (an angular velocity sensor and an acceleration sensor), a control unit that controls the detection by the sensors, and a memory that stores detection results of the sensors. The sensing devices 50 are attached to both ankles and the waist of a patient when the patient conducts a gait test, which is one of the motor function tests, under the direction of a physical therapist, and detect temporal variations in angular velocity and acceleration. The sensing device 50 also includes an input button for inputting the start of the measurement and the end of the measurement.

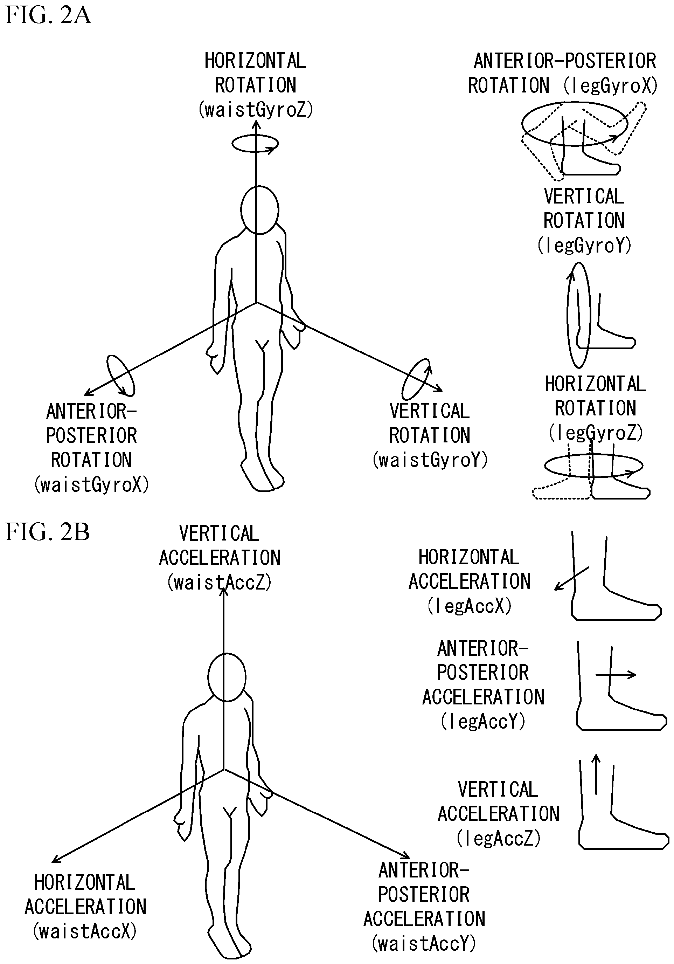

FIG. 2A and FIG. 2B illustrate types of data acquired from the sensing devices 50 attached to legs and a waist. FIG. 2A illustrates types of data acquired from the angular velocity sensor, and FIG. 2B illustrates types of data acquired from the acceleration sensor.

As illustrated in the left part of FIG. 2A, data on anterior-posterior rotation (waistGyroX), data on vertical rotation (waistGyroY), and data on horizontal rotation (waistGyroZ) are acquired from the angular velocity sensor attached to the waist. As illustrated in the right part of FIG. 2A, data on anterior-posterior rotation (legGyroX), data on vertical rotation (legGyroY), and data on horizontal rotation (legGyroZ) are acquired from the angular velocity sensor attached to the ankle.

Additionally, as illustrated in the left part of FIG. 2B, data on horizontal acceleration (waistAccX), data on anterior-posterior acceleration (waistAccY), and data on vertical acceleration (waistAccZ) are acquired from the acceleration sensor attached to the waist. As illustrated in the right part of FIG. 2B, data on horizontal acceleration (legAccX), data on anterior-posterior acceleration (legAccY), and data on vertical acceleration (legAccZ) are acquired from the acceleration sensor attached to the ankle.

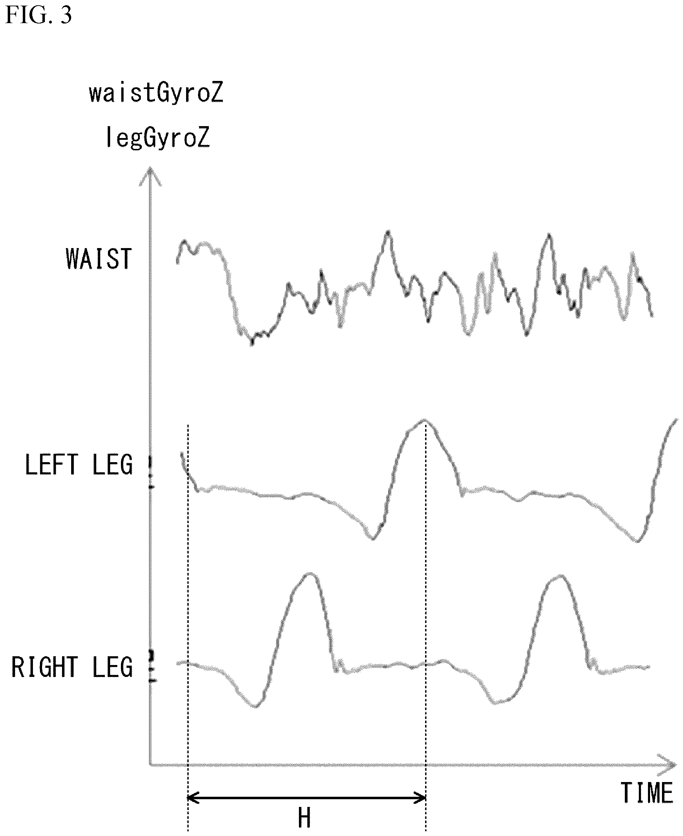

FIG. 3 illustrates, as an example, time-series data of the horizontal rotation (waistGyroZ) acquired from the angular velocity sensor attached to the waist and time-series data of the horizontal rotation (legGyroZ) acquired from each of the angular velocity sensors attached to the ankles.

Referring back to FIG. 1, the physical therapist terminal 60 is a terminal such as a personal computer (PC) or a tablet terminal used by the physical therapist. The physical therapist terminal 60 acquires detection results (time-series data of angular velocities and accelerations) of the sensing devices 50 by connecting to the sensing devices 50. Additionally, the physical therapist terminal 60 transmits the acquired time-series data of the angular velocities and the accelerations to the server 10 through the network 80.

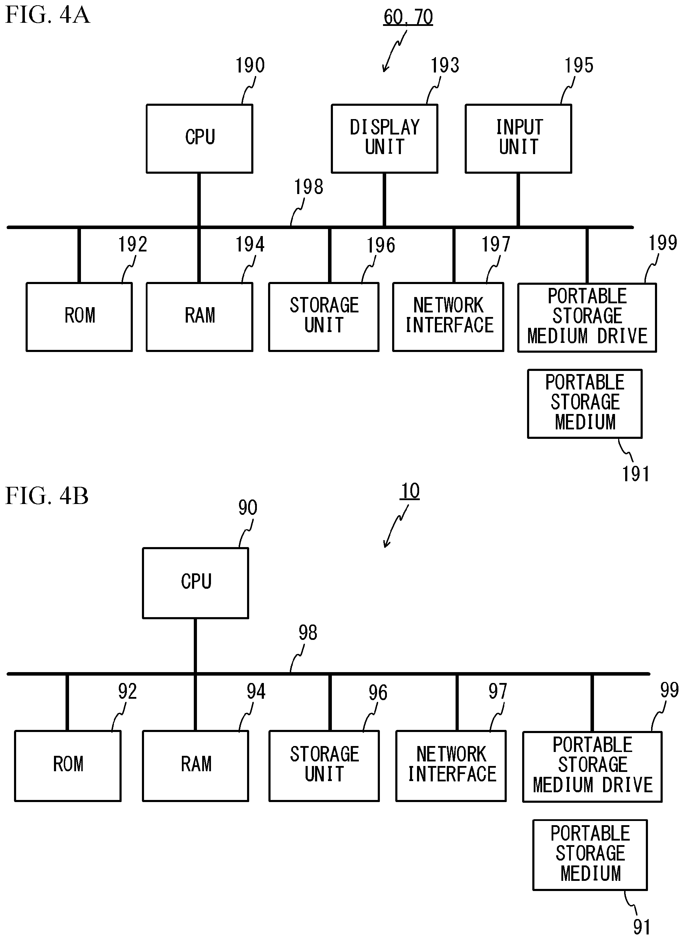

FIG. 4A illustrates a hardware configuration of the physical therapist terminal 60. As illustrated in FIG. 4A, the physical therapist terminal 60 includes a central processing unit (CPU) 190, a read only memory (ROM) 192, a random access memory (RAM) 194, a storage unit (e.g., a solid state drive (SSD) or a hard disk drive (HDD)) 196, a network interface 197, a display unit 193, an input unit 195, and a portable storage medium drive 199. The display unit 193 includes a liquid crystal display or the like, and the input unit 195 includes a keyboard, a mouse, a touch panel, or any combination of them. The portable storage medium drive 199 is a device that can read data stored in a portable storage medium 191. These components of the physical therapist terminal 60 are coupled to a bus 198. The physical therapist terminal 60 also includes a communication unit that communicates with the sensing devices 50 wirelessly or by wire.

Referring back to FIG. 1, the doctor terminal 70 is a terminal such as a PC or the like used by a doctor. The doctor terminal 70 displays information transmitted from the server 10 to present the information to the doctor, and has the same hardware configuration as the physical therapist terminal 60 as illustrated in FIG. 4A. The doctor refers to the information displayed on the doctor terminal 70 to check symptoms of the patient, consider a course of treatment, and check medication effectiveness.

The server 10 analyzes the movement corresponding to the symptom of the patient based on the time-series data of the angular velocities and the accelerations acquired from the physical therapist terminal 60. The server 10 make a determination regarding the gait state of the patient, and outputs a screen presenting the determination result to the doctor terminal 70. More specifically, the server 10 calculates the feature amount of a first movement (a compensating movement) corresponding to the symptom (the disease name) of the patient and the feature amount of a second movement (a compensated movement) correlating with the compensating movement, based on the time-series data of the angular velocities and the accelerations. Then, the server 10 makes a determination regarding the gait state of the patient based on the calculated feature amounts. Here, the compensating movement means a movement in which another part of the body achieves the aimed motion instead of the diseased part to assist the insufficiency of the aimed motion. Examples of the compensating movement include, but are not limited to, starting to walk using the body trunk without bending the legs and walking with a pigeon-toe gait. The compensated movement means another movement that correlates with the compensating movement and appears immediately before or immediately after the compensating movement. Examples of the compensated movement include, but are not limited to, causing the heel to come in contact with the ground, lifting the toe from the ground, and moving the waist.

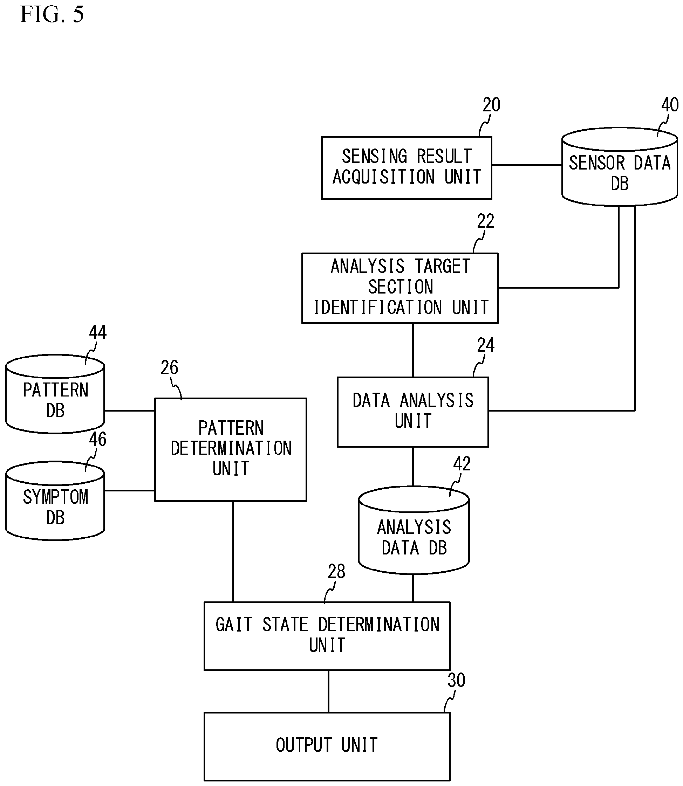

FIG. 4B illustrates a hardware configuration of the server 10. The server 10 includes a CPU 90, a ROM 92, a RAM 94, a storage unit (an SSD or an HDD) 96, a network interface 97, and a portable storage medium drive 99. These components of the server 10 are coupled to a bus 98. In the server 10, the functions of each unit illustrated in FIG. 5 are implemented by the CPU 90 executing programs (including a gait state determination program) stored in the ROM 92 or the storage unit 96, or programs read by the portable storage medium drive 99 from the portable storage medium 91. The functions of each unit in FIG. 5 may be implemented by an integrated circuit such as, but not limited to, an application specific integrated circuit (ASIC) or a field programmable gate array (FPGA).

FIG. 5 is a functional block diagram of the server 10. In the server 10, execution of the programs by the CPU 90 implements the functions as a sensing result acquisition unit 20, an analysis target section identification unit 22, a data analysis unit 24, a pattern determination unit 26, a gait state determination unit 28, and an output unit 30.

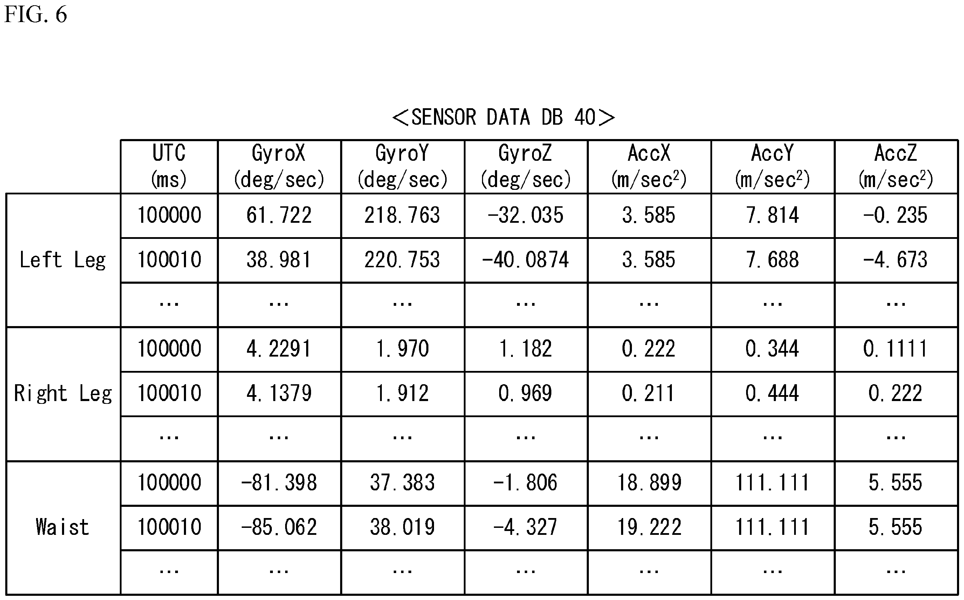

The sensing result acquisition unit 20 acquires data detected by the sensing devices 50 during the gait test of the patient, and stores the acquired data in a sensor data database (DB) 40. Here, the sensor data DB 40 has a data structure illustrated in FIG. 6, as an example. The sensor data DB 40 stores data acquired in the angular velocity sensors and the acceleration sensors at each time (UTC) as illustrated in FIG. 6. FIG. 3 is obtained by illustrating data (GyroZ) indicating the horizontal rotations acquired from the acceleration sensors attached to the waist and both ankles graphically among data presented in FIG. 6.

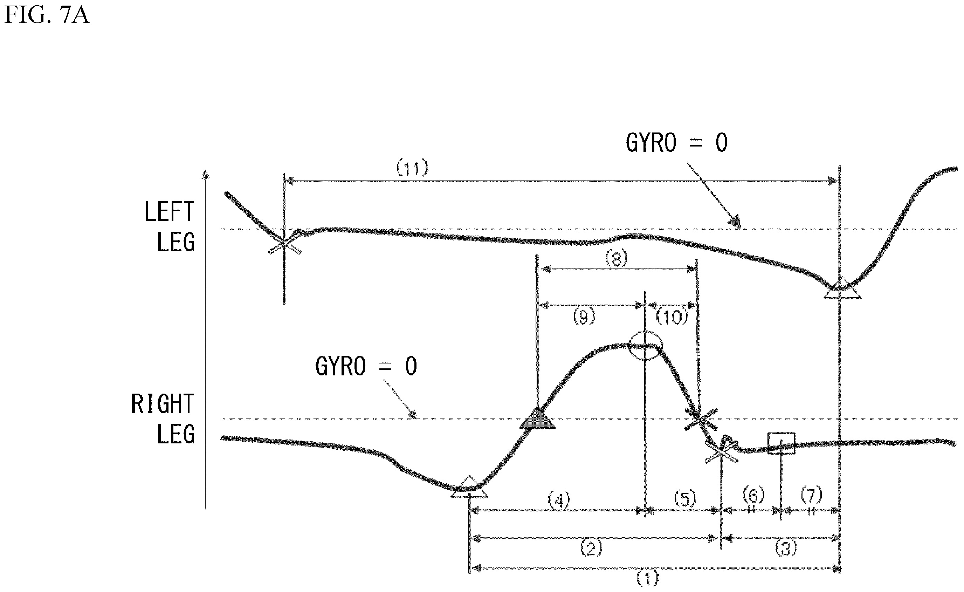

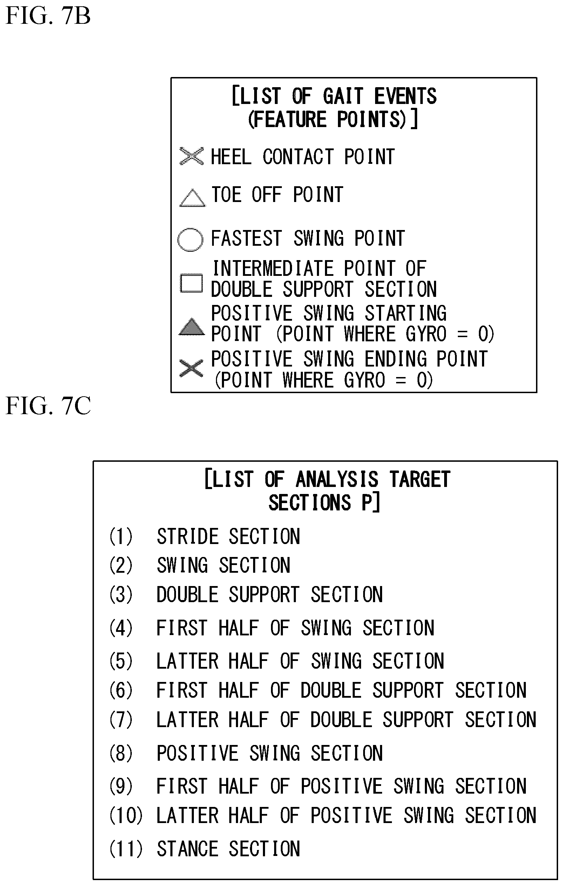

The analysis target section identification unit 22 extracts gait events (feature points) from the time-series data acquired through one gait test. The analysis target section identification unit 22 uses the data (e.g., the time-series data of the horizontal rotations (legGyroZ)) acquired from both ankles in FIG. 3 when extracting the gait events. FIG. 7A is an enlarged view of part (an area H) of the time-series data of the left leg and the right leg illustrated in FIG. 3. FIG. 7B presents a list of the gait events (the feature points). The analysis target section identification unit 22 analyzes the data illustrated in FIG. 7A to identify the gait events (the feature points) from the time-series data. The analysis target section identification unit 22 identifies each gait event (the feature point). For example, the analysis target section identification unit 22 identifies the timing when the value of the angular velocity is on a rising trend and the value of the angular velocity becomes 0 (gyro = 0) in the time-series data of the right leg as the "positive swing starting point". Then, the analysis target section identification unit 22 identifies 11 analysis target sections P based on respective gait events (the feature points). Here, the analysis target sections include sections illustrated in FIG. 7C. For example, the stride section is defined as the section between the toe off point of one leg and the toe off point of another leg. Thus, the section indicated by (1) in FIG. 7A is identified as the stride section. Additionally, the swing section is defined as the section between the toe off point of one leg and the heel contact point of the same leg. Thus, the section indicated by (2) in FIG. 7A is identified as the swing section. In the similar manner, each gait section is identified. The numbers in parentheses in FIG. 7A correspond to the numbers in parentheses in FIG. 7C.

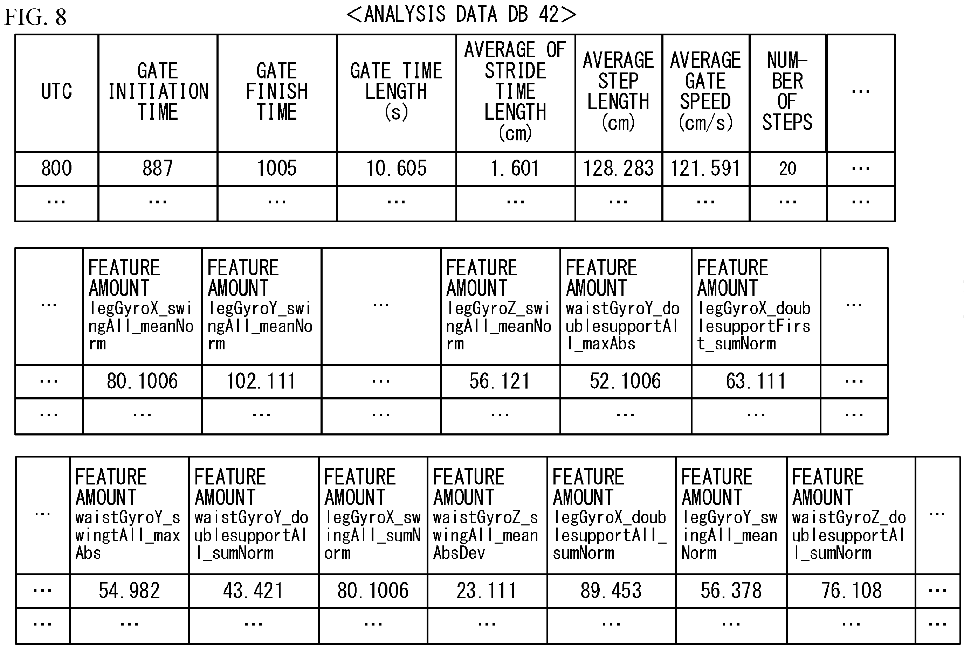

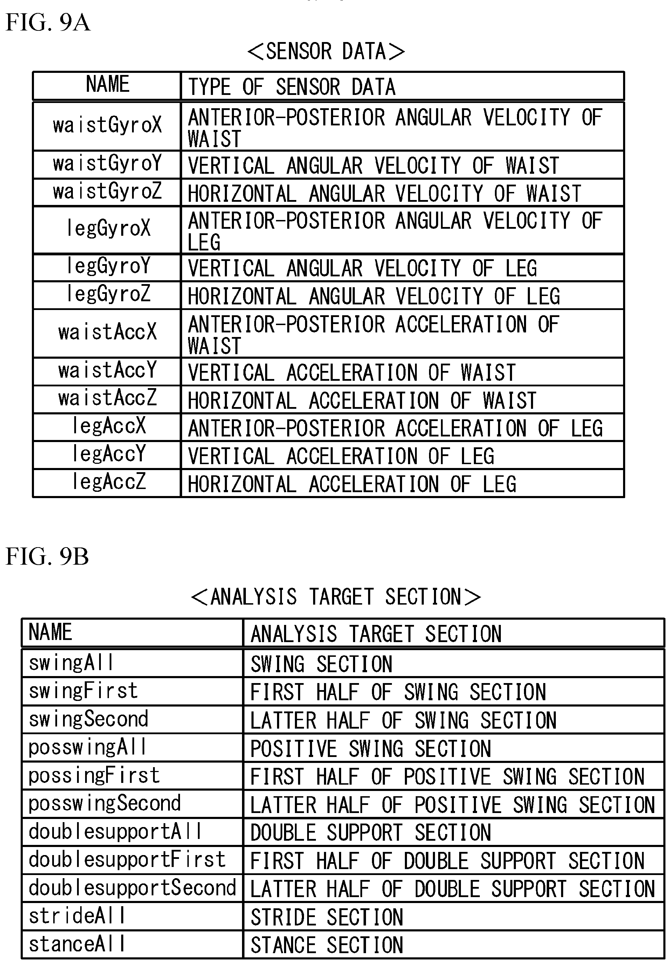

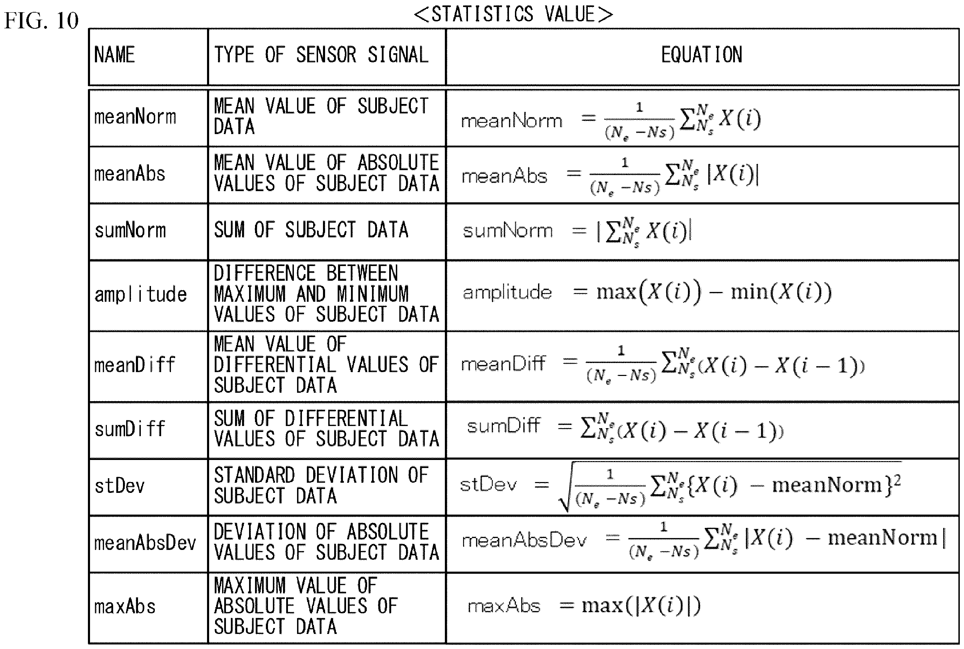

The data analysis unit 24 analyzes the time-series data using the analysis target sections identified by the analysis target section identification unit 22 to acquire information on gate and feature amounts, and stores them in an analysis data DB 42. The analysis data DB 42 has a data structure illustrated in FIG. 8. The analysis data DB 42 stores the "information on gate" and the "feature amount" in association with the time (UTC) when the gait test is started. The information on gait includes information such as the "gate initiation time", the "gait finish time", the "gait time length (s)", the "average of stride time length (s)", the "average step length (cm)", the "average gate speed (cm/s)", and the "number of steps". Additionally, the feature amounts include various statistics values of the data of each sensor in each of the analysis target sections. The feature amount in FIG. 8 is described in the form of "sensor data analysis target section_statistics value" that indicates the sensor data, the analysis target section, and the statistics value in this order. The sensor data includes 12 types of data as illustrated in FIG. 9A, and the analysis target sections include 11 types of analysis target sections as illustrated in FIG. 9B. The statistics values include 9 types of statistics values as illustrated in FIG. 10. The equation of each statistics value in FIG. 10 is the equation when the sensor signal X(i) (i ∈ [Ns, Ne]) is acquired where $N_s$ represents the starting point and Ne represents the ending point. For example, among the anterior-posterior angular velocities of the waist, the mean value of data in the swing section is described as "waistGyroX_swingAll_meanNorm". In the case of FIG. 8, there are 1188 (= 12 × 11 × 9) types of feature amounts in actuality.

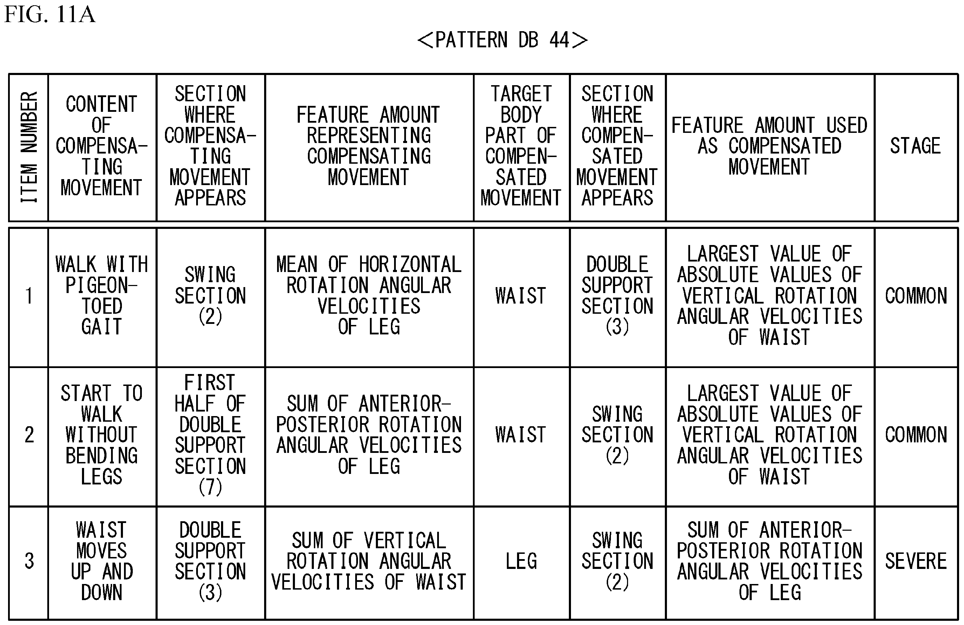

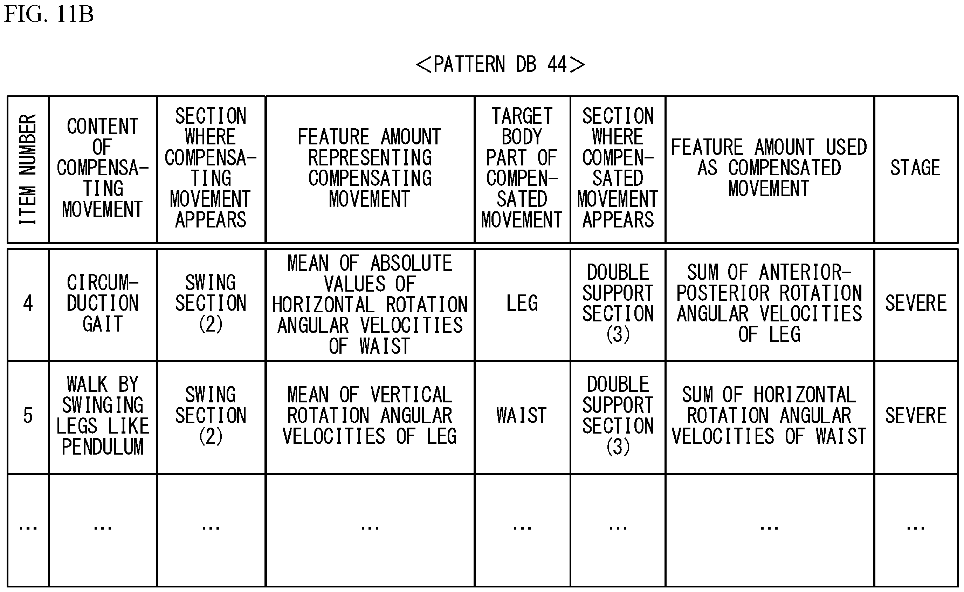

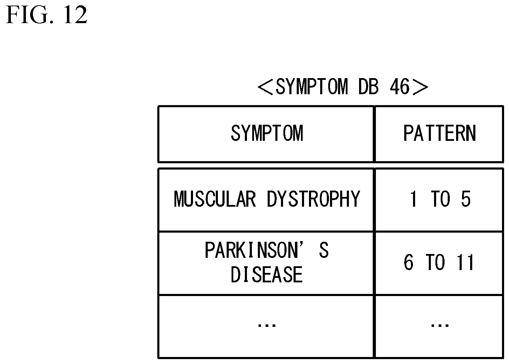

The pattern determination unit 26 determines the pattern of the combination of the compensating movement and the compensated movement according to the symptom of the patient. When determining the pattern, the pattern determination unit 26 refers to a pattern DB 44 (FIG. 11A and 11B) and a symptom DB 46 (FIG. 12). The pattern DB 44 stores information on the combination of the compensating movement and the compensated movement. More specifically, the pattern DB 44 stores the "content of compensating movement", the "section where compensating movement appears", the "feature amount representing compensating movement", the "target body part of compensated movement", the "section where compensated movement appears", the "feature amount used as compensated movement", and the "stage" in association with the item number as illustrated in FIG. 11A and FIG. 11B. The "content of compensating movement" indicates the specific content of the compensating movement, the "section where compensating movement appears" indicates in which of 11 analysis target sections the compensating movement appears. The "feature amount representing compensating movement" indicates which of the feature amounts presented in FIG. 8 is to be used as the feature amount of the compensating movement. The "target body part of compensated movement" indicates the part of the body where the compensated movement appears, and the "section where compensated movement appears" indicates the analysis target section where the compensated movement appears. The "feature amount used as compensated movement" indicates which of the feature amounts presented in FIG. 8 is to be used as the feature amount of the compensated movement. "Common" or "severe" is described in the "stage". A pattern described as "common" means a pattern used to determine the presence or absence of a symptom, and a pattern described as "severe" means a pattern used to determine whether the symptom is severe. The "section where compensating movement appears" is an example of a second period during which the first movement is performed. The "feature amount representing compensating movement" is an example of a third feature amount indicating the first movement. The "section where compensated movement appears" is an example of a third period during which the second movement is performed. The "feature amount used as compensated movement" is an example of a fourth feature amount indicating the second movement.

The symptom DB 46 stores the symptom and the item number of the pattern DB 44 in association with each other as illustrated in FIG. 12. For example, when the patient is suffering from muscular dystrophy, the pattern determination unit 26 refers to the symptom DB 46, identifies the item numbers (1 to 5) corresponding to muscular dystrophy, and sets the patterns corresponding to the identified item numbers (1 to 5) in the pattern DB 44 illustrated in FIG. 11A and FIG. 11B as the patterns used for the process.

The gait state determination unit 28 acquires the feature amount of the compensating movement and the feature amount of the compensated movement with respect to each of the patterns set by the pattern determination unit 26, and calculates the compensation change using the acquired feature amounts. The gait state determination unit 28 also determines the gait state of the patient, i.e., the presence or absence of the symptom and the severity based on the calculated compensation change.

The output unit 30 acquires the result determined by the gait state determination unit 28, generates an output screen, and outputs the output screen to the doctor terminal 70. The doctor terminal 70 acquires the output screen, and displays the output screen on the display unit 193.

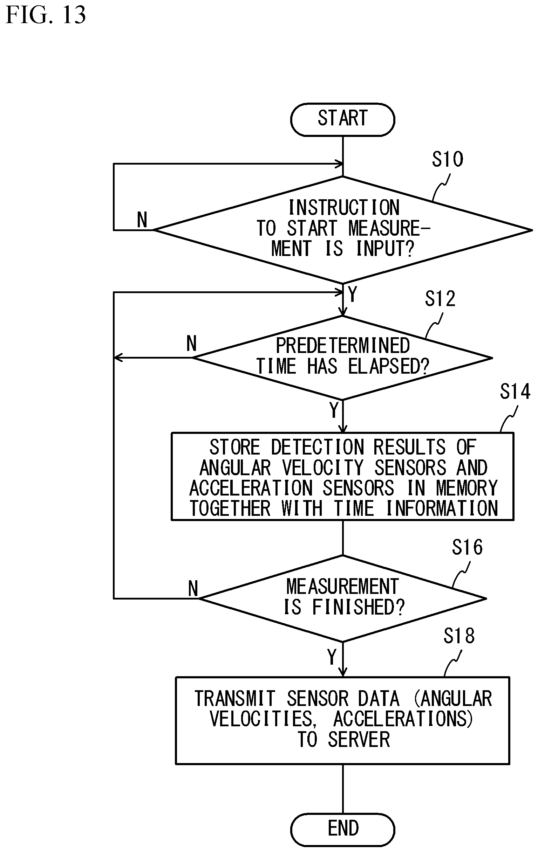

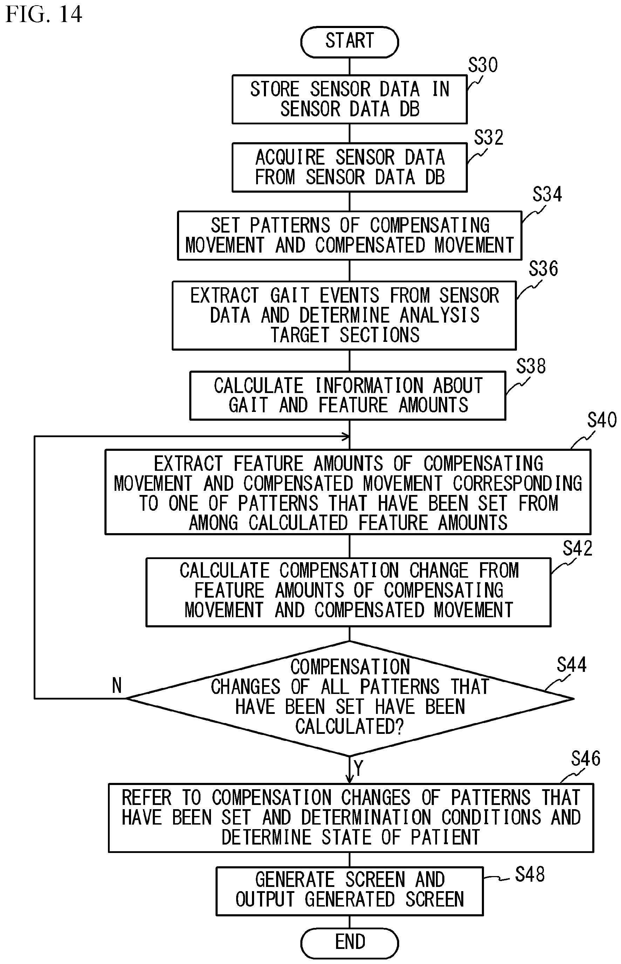

Next, a description will be given of a pre-process executed by the sensing device 50 and a data analysis process executed by the server 10 along the flowcharts illustrated in FIG. 13 and FIG. 14.

(Pre-process executed by the sensing device 50)

FIG. 13 is a flowchart illustrating the pre-process executed by the sensing device 50. In this pre-process, the sensing device 50 acquires the time-series data of the angular velocity and the acceleration during the period when the patient is conducting the gait test under the direction of the physical therapist. As a precondition to the process of FIG. 13, the patient wears the sensing devices 50 on both ankles and the waist, and is ready for the gait test. The gait test is conducted for a distance of, for example, 10 m, and the sensing devices 50 detect the angular velocities and the accelerations of both ankles and the waist while the patient is walking straight for a distance of 10 m.

In the process of FIG. 13, in step S10, the control unit of the sensing device 50 waits until an instruction to start measurement is input. For example, when an input button, which is provided to the sensing device 50 and is used to input the start of the measurement, is pressed by a physical therapist, the control unit proceeds to step S12.

In step S12, the control unit waits until a predetermined time elapses. The predetermined time here means the measurement interval, and is about several milliseconds to several tens of milliseconds.

When the predetermine time has elapsed and the process proceeds to step S14, the control unit detects the angular velocities and the accelerations using the angular velocity sensors and the acceleration sensors, and stores them in the memory together with time information.

Then, in step S16, the control unit determines whether the measurement is finished. For example, when the input button for inputting the end of measurement is pressed by the physical therapist, the determination in step S16 becomes YES. By contrast, when the input button is not pressed, the process returns to step S12. Thereafter, until the determination in step S16 becomes YES, the control unit repeats the processes and the determinations in steps S12 to S16. When the determination in step S16 becomes YES, the process proceeds to step S18, and the control unit transmits the data stored in the memory to the physical therapist terminal 60 and ends the entire process of FIG. 13. The physical therapist relates the data to the patient in the physical therapist terminal 60, and then transmits the data to the server 10.

(Data analysis process executed by the server 10)

Next, the data analysis process executed by the server 10 will be described based on FIG. 14. The process of FIG. 14 is a process started when the time-series data of the angular velocity and the acceleration of a certain patient (a subject patient) is transmitted from the physical therapist terminal 60, as an example.

In the process of FIG. 14, in step S30, the sensing result acquisition unit 20 acquires the time-series data (the sensor data) of the angular velocities and the accelerations of the subject patient, and stores them in the sensor data DB 40. Here, the sensing result acquisition unit 20 acquires the data such as the data illustrated in FIG. 3, and stores the acquired data in the sensor data DB 40 illustrated in FIG. 6.

Then, in step S32, the analysis target section identification unit 22 acquires the data stored in the sensor data DB 40.

Then, in step S34, the analysis target section identification unit 22 acquires the symptom of the subject patient from an electronic health record server or the like, and identifies the item number of the pattern corresponding to the symptom of the subject patient based on the symptom DB 46 of FIG. 12. The analysis target section identification unit 22 refers to the pattern DB 44 illustrated in FIG. 11A and FIG. 11B, extracts the pattern of the compensating movement and the compensated movement corresponding to the identified item number. For example, when the symptom of the subject patient is muscular dystrophy, the patterns of the item numbers 1 to 5 are set as the patterns to be used, based on FIG. 12.

Then, in step S36, the analysis target section identification unit 22 extracts the gait events (the feature points) from the sensor data and determines the analysis target sections. Here, the analysis target section identification unit 22 extracts the gait event for each step of the patient as illustrated in FIG. 7A, and determines the analysis target sections for each step of the patient.

Then, in step S38, the data analysis unit 24 calculates the information on gait and the feature amounts to be stored in the analysis data DB 42 illustrated in FIG. 8. The information on gait is information such as the gait time length, the average of the stride time length, and the like presented in FIG. 8, and the feature amounts are 1188 types of feature amounts such as "legGyroX_swingAll_meanNorm".

Then, in step S40, the gait state determination unit 28 extracts the feature amount $D(Xm)$ of the compensating movement and the feature amount $D(Ym)$ of the compensated movement corresponding to one (for example, a pattern m) of the patterns that have been set, among the calculated feature amounts. For example, in the case of the pattern of the item number 1 in the pattern DB 44 illustrated in FIG. 11A, the gait state determination unit 28 extracts the value (56.121) of "legGyroZ_swingAllmeanNorm" as the feature amount $D(Xm)$. The gait state determination unit 28 also extracts the value (52.1006) of "waistGyroY_doublesupportAll_maxAbs" as the feature amount $D(Ym)$.

Then, in step S42, the gait state determination unit 28 calculates the compensation change $\Delta Dm$ from the following equation (1) using the feature amount $D(Xm)$ of the compensating movement and the feature amount $D(Ym)$ of the compensated movement.

$$\Delta Dm = D(Xm) / D(Ym) \qquad\qquad (1)$$

Then, in step S44, the gait state determination unit 28 determines whether the compensation changes of all the patterns that have been set have been calculated. When the determination in step S44 is NO, the process returns to step S40, and the process of extracting the feature amounts of the compensating movement and the compensated movement (S40) and the process of calculating the compensation change (S42) are repeated. When the determination in step S44 becomes YES, the process proceeds to step S46.

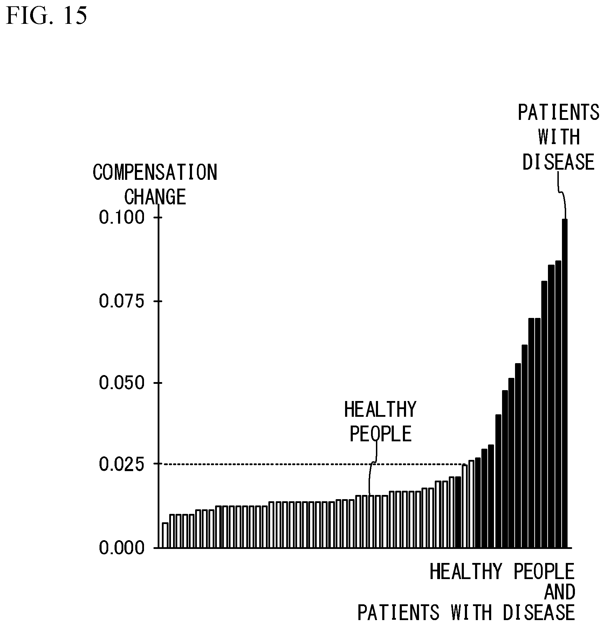

In step S46, the gait state determination unit 28 refers to the compensation changes of the patterns that have been set and the determination condition to determine the state of the patient. For example, discussed is a case where the compensation changes of healthy people and the compensation changes of patients with diseases are distributed as illustrated in FIG. 15 as a result of the examination conducted in advance in the case of the pattern of the item number 2 in the pattern DB 44 illustrated in FIG. 11A. The distribution presented in FIG. 15 reveals that the boundary between the healthy people and the patients with diseases is at a compensation change of ≈ 0.025. Therefore, the gait state determination unit 28 determines that the patient experiences the symptom when the compensation change is 0.025 or greater for the pattern of the item number 2. In the similar manner, the gait state determination unit 28 checks whether the compensation changes are included within the respective ranges determined with respect to the patterns of which the stages are "common" in the pattern DB 44 illustrated in FIG. 11A and FIG. 11B, and determines that the patient experiences the symptom when any of the compensation changes is included in the corresponding range.

Additionally, in the similar manner, it is checked whether the compensation changes are included in the respective ranges predetermined with respect to the patterns of which the stages are "severe", and the severity is determined based on the ratio of the patterns of which the compensation changes are included in the respective predetermined ranges to all the patterns of which the stages are "severe". For example, when there are three patterns of which the stages are "severe", and one of the compensation changes corresponding the three patterns is included in the predetermined range, the gait state determination unit 28 determines that the severity is 33.3%.

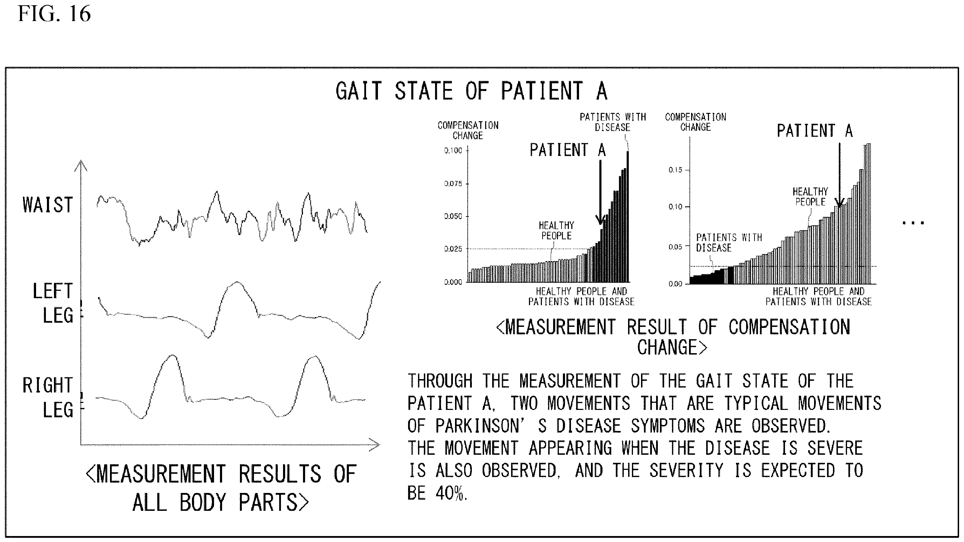

Then, in step S48, the output unit 30 generates a screen based on the determination result of step S46, and outputs the generated screen to the doctor terminal 70. The screen generated by the output unit 30 is, for example, a screen illustrated in FIG. 16. The screen illustrated in FIG. 16 displays the measurement results of the sensors of the ankles and the waist (the measurement results of all body parts), the measurement result of the compensation change, and textual information indicating the presence or absence of the symptom and the severity. The output unit 30 collects necessary data from FIG. 6 based on the UTC of the data (the data in the analysis data DB 42 (FIG. 8)) that have been used for analysis, the gate initiation time, and the gait finish time when displaying the measurement results of all body parts. In FIG. 16, the measurement results of the angular velocity sensors or the acceleration sensors in one direction are presented in the space for the measurement results of all body parts. However, this does not intend to suggest any limitation, and the measurement results of the angular velocity sensors or the acceleration sensors in different directions may be presented next to each other. The output unit 30 may generate a screen that can display the measurement results of the angular velocity sensors or the acceleration sensors in the directions selected by the doctor according to the selection by the doctor.

As is clear from the above description, in the present embodiment, step S30 achieves the process of acquiring the data of the movement of at least a part of the body during the period when the patient is walking, using the sensing device 50 attached to the body of the patient. Steps S34, S36, S38, and S40 achieve the process of identifying a first feature amount of a predetermined first movement (the compensating movement) corresponding to the symptom of the patient and a second feature amount of a second movement (the compensated movement) correlating with the first movement (the compensating movement), from the data acquired from the sensing device 50. Further, steps S42, S46, and S48 achieve the process of making a determination regarding the gait state of the patient based on the feature amount of the first movement (the compensating movement) and the feature amount of the second movement (the compensated movement) and outputting the determination result.

As described above in detail, in the present embodiment, the server 10 acquires the time-series data of the angular velocities and the accelerations of the ankles and the waist during the period when the patient conducts the gait test, which are detected using the sensing devices 50 attached to the body of the patient. In addition, the server 10 identifies the feature amount of the predetermined compensating movement corresponding to the symptom of the patient and the feature amount of the compensated movement correlating with the compensating movement from the detected time-series data. Then, the server 10 determines the presence or absence of the symptom of the patient and the severity based on the ratio of the feature amount of the compensating movement and the feature amount of the compensated movement (the compensation change), and outputs the determination result. This allows the present embodiment to determine the presence or absence of the symptom and the severity accurately because the present embodiment quantifies the characteristic movements that appear in gait for each disease, and determines the presence or absence of the symptom and the severity based on the value. Therefore, the doctor can check the symptom of the patient, consider a course of treatment, and check medication effectiveness by referring to the information displayed on the doctor terminal 70. In this case, by performing a determination using the compensation change of the pattern selected according to the symptom, more precise determination of the symptom can be made than in the case using the conventional classification of disability stages (see https://www.neurology-jp.org/guidelinem/pdf/dmd_03.pdf, for example).

In addition, in the present embodiment, it is defined in advance in the pattern DB 44 that the feature amount of the compensating movement and the feature amount of the compensated movement are what kind of statistics value detected by which sensor during which analysis target section. This allows the gait state determination unit 28 to easily identify the feature amount of the compensating movement and the feature amount of the compensated movement.

Additionally, in the present embodiment, the gait state determination unit 28 determines the presence or absence of the symptom based on whether the ratio of the feature amount of the compensating movement and the feature amount of the compensated movement (the compensation change) is included in a predetermined range. This allows the presence or absence of the symptom to be accurately determined based on the feature amount of the compensating movement and the feature amount of the compensated movement determined with respect to each symptom.

In addition, in the present embodiment, in the pattern DB 44, the pattern used to determine the presence or absence of the symptom (stage = "common") and the pattern used to determine the severity (stage = "severe") are prepared. Therefore, the presence or absence of the symptom and the severity can be accurately determined.

In addition, in the present embodiment, the patient wears the sensing devices 50 on the ankles and the waist when conducting the gait test. This prevents muscles and thick blood vessels from being compressed unlike in the case where the sensing device 50 is attached to the thigh. This reduces the physical load on the patient.

The above embodiment describes a case where the data analysis unit 24 calculates all 1188 feature amounts in step S38 of FIG. 14, but does not intend to suggest any limitation. For example, the data analysis unit 24 calculates only the feature amounts included in the patterns that are determined, by the pattern determination unit 26, to be used, and store the calculated feature amounts in the analysis data DB 42.

The above embodiment describes a case where the pattern used to determine the presence or absence of the symptom is different from the pattern used to determine the severity, but does not intend to suggest any limitation. For example, a plurality of threshold values may be set for each pattern to divide the compensation change into ranges such as "normal", "mild", and "severe", and the severity of the patient may be determined based on which range the compensation change of the patient falls within. For example, when the compensation change is calculated for N patterns for one patient, it is checked and counted which of "normal", "mild", and "severe" each compensation change indicates. Then, the severity that are most counted may be determined as the severity of the patient among "normal", "mild", and "severe". Alternatively, each pattern may be weighed, and the severity of the patient may be determined taking into account the weight.

In the above embodiment, in the pattern DB 44 illustrated in FIGs. 11A and 11B, the pattern in which the target body part of the compensating movement is different from the target body part of the compensated movement, but this does not intend to suggest any limitation. The target body part of the compensating movement may be the same as the target body part of the compensated movement.

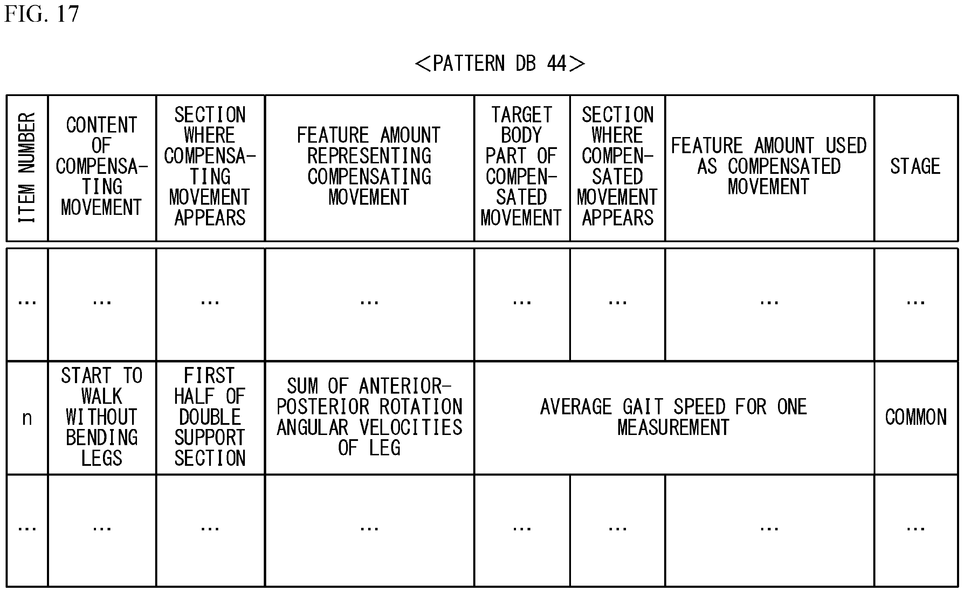

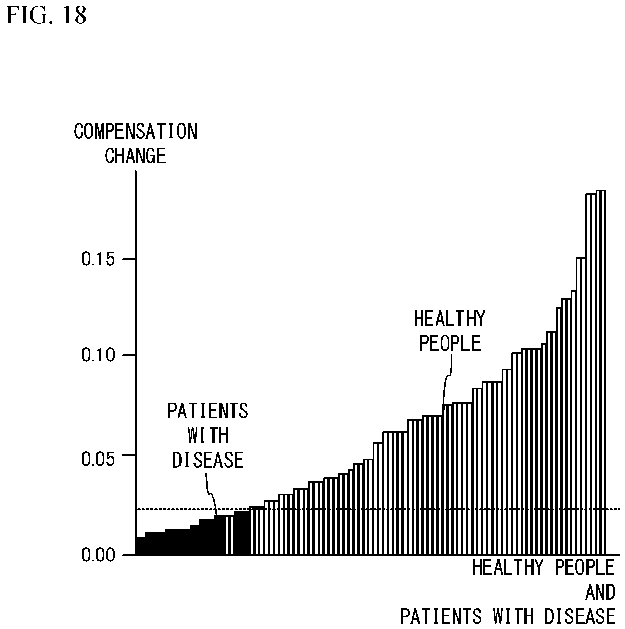

The above embodiment describes a case where the movement correlating with the compensating movement is the compensated movement before or after the compensating movement, but does not intend to suggest any limitation. The movement correlating with the compensating movement may not be necessarily the compensated movement. For example, as indicated by the item number n in FIG. 17, instead of the compensated movement, the feature amount relating to gait such as the "average gait speed for one measurement" may be used. Discussed is a case where in the pre-examination, the compensation changes of healthy people and the compensation changes of patients with diseases are distributed as illustrated in FIG. 18 in this case. From the distribution illustrated in FIG. 18, it is considerable that the boundary between the patients with diseases and the healthy people is at a compensation change of ≈ 0.025. Therefore, the gait state determination unit 28 determines that the patient experiences the symptom when the compensation change is 0.025 or less for the pattern of the item number n.

The above embodiment determines the gait state (the presence or absence of symptoms and the severity) of the patient based on the compensation change acquired from the ratio of the feature amount of the compensating movement and the feature amount of the compensated movement (see the equation (1)), but does not intend to suggest any limitation. For example, the value acquired from the feature amount of the compensation change and the feature amount of the compensated movement may be defined as the compensation change, and the gait state of the patient may be determined based on this value.

The above embodiment describes a case where the sensing device 50 includes both the angular velocity sensor and the acceleration sensor, but does not intend to suggest any limitation. The sensing device 50 may include only one of the angular velocity sensor and the acceleration sensor.

The above embodiment describes a case where the process of FIG. 14 is executed by the server 10, but does not intend to suggest any limitation. For example, part of or the entire of the process in FIG. 14 may be executed by the physical therapist terminal 60 or the doctor terminal 70. That is, the process of FIG. 14 is achieved by one or more devices in the information processing system 100.

The above embodiment describes a case where the sensing devices 50 are connected to the physical therapist terminal 60, but does not intend to suggest any limitation. The sensing device 50 may be connected to the doctor terminal 70. In this case, the doctor terminal 70 executes the same process as the physical therapist terminal 60 in the above embodiment.

The above embodiment describes a case where the output unit 30 outputs the feature amounts to the doctor terminal 70, but does not intend to suggest any limitation. The output unit 30 may output the feature amounts to other terminals. For example, the output unit 30 may output the feature amounts to the physical therapist terminal 60 or the terminal of a pharmaceutical company conducting trials.

The above-described processing functions are implemented by a computer. In this case, a program in which processing details of the functions that a processing device (CPU) is to have are written is provided. The aforementioned processing functions are implemented by the computer executing the program. The program in which the processing details are written can be stored in a computer-readable storage medium (however, excluding carrier waves).

When the program is distributed, it may be sold in the form of a portable storage medium such as a digital versatile disc (DVD) or a compact disc read only memory (CD-ROM) storing the program. The program may be stored in a storage device of a server computer, and the program may be transferred from the server computer to another computer over a network.

A computer executing the program stores the program stored in a portable storage medium or transferred from a server computer in its own storage device. The computer then reads the program from its own storage device, and executes a process according to the program. The computer may directly read the program from a portable storage medium, and execute a process according to the program. Alternatively, the computer may successively execute a process, every time the program is transferred from a server computer, according to the received program.

Although the exemplary embodiments of the present invention have been illustrated in detail, the present invention is not limited to the above-mentioned embodiments, and other embodiments, variations and modifications may be made without departing from the scope of the present invention.

**Claims**

1. A gait state determination system comprising:

   a sensor (50) attached to a body of a person; and
   an information processing device (10) configured to:

   acquire, using the sensor, data of a movement of at least a part of the body during a first period when the person is walking,
   identify a first feature amount of a first movement from the data acquired, and identify a second feature amount of a second movement from the data acquired, the first movement being determined in advance and corresponding to a symptom of the person, the second movement correlating with the first movement,
   make a determination regarding a gait state of the person based on the first feature amount of the first movement and the second feature amount of the second movement, and
   output a result of the determination.

2. The gait state determination system according to claim 1,

   wherein a second period during which the first movement is performed, a third period during which the second movement is performed during gait, a third feature amount indicating the first movement, and a fourth feature amount indicating the second movement are determined in advance, and
   wherein the information processing device (10) identifies the third feature amount and the fourth feature amount from the data acquired during the second period and during the third period.

3. The gait state determination system according to claim 1 or 2, wherein the information processing device (10) make a determination regarding the symptom based on whether a ratio of the first feature amount of the first movement and the second feature amount of the second movement is included within a predetermined range.

4. The gait state determination system according to any one of claims 1 to 3, the determination regarding the gait state of the person includes a determination regarding presence or absence of the symptom or a determination regarding a progression of the symptom.

5. The gait state determination system according to any one of claims 1 to 4, wherein the first feature amount of the first movement and the second feature amount of the second movement are identified from data of movements of different parts of the body.

6. The gait state determination system according to any one of claims 1 to 4, wherein the first feature amount of the first movement and the second feature amount of the second movement are identified from data of a movement of a same part of the body.

7. The gait state determination system according to any one of claims 1 to 6, wherein the sensor (50) is attached to at least an ankle of the person and a waist of the person.

8. The gait state determination system according to any one of claims 1 to 7, wherein the second movement is a movement conducted by the person at one or both of following timings: before the first movement and after the first movement.

9. The gait state determination system according to any one of claims 1 to 8, wherein the sensor includes an angular velocity sensor, an acceleration sensor, or both the angular velocity sensor and the acceleration sensor.

10. A gait state determination method implemented by a computer, the gait state determination method comprising:

acquiring data of a movement of at least a part of a body of a person during a period when the person is walking, using a sensor attached to the body of the person (S30);
identifying a first feature amount of a first movement from the data acquired, and a second feature amount of a second movement from the data acquired, the first movement being determined in advance and corresponding to a symptom of the person, the second movement correlating with the first movement (S34, S36, S38, S40);
making a determination regarding a gait state of the person based on the first feature amount of the first movement and the second feature amount of the second movement (S42, S46); and
outputting a result of the determination (S48).

11. A gait state determination program that causes a computer to execute a process, the process comprising:

acquiring data of a movement of at least a part of a body of a person during a period when the person is walking, using a sensor attached to the body of the person (S30);
identifying a first feature amount of a first movement from the data acquired, and a second feature amount of a second movement from the data acquired, the first movement being determined in advance and corresponding to a symptom of the person, the second movement correlating with the first movement (S34, S36, S38, S40);
making a determination regarding a gait state of the person based on the first feature amount of the first movement and the second feature amount of the second movement (S42, S46); and
outputting a result of the determination (S48).

FIG. 1

FIG. 2A

HORIZONTAL
ROTATION
(waistGyroZ)

ANTERIOR-POSTERIOR
ROTATION (legGyroX)

VERTICAL
ROTATION
(legGyroY)

ANTERIOR-
POSTERIOR
ROTATION
(waistGyroX)

VERTICAL
ROTATION
(waistGyroY)

HORIZONTAL
ROTATION
(legGyroZ)

FIG. 2B

VERTICAL
ACCELERATION
(waistAccZ)

HORIZONTAL
ACCELERATION
(legAccX)

ANTERIOR-
POSTERIOR
ACCELERATION
(legAccY)

VERTICAL
ACCELERATION
(legAccZ)

HORIZONTAL
ACCELERATION
(waistAccX)

ANTERIOR-
POSTERIOR
ACCELERATION
(waistAccY)

FIG. 3

waistGyroZ
legGyroZ

WAIST

LEFT LEG

RIGHT LEG

H

TIME

FIG. 4A

60, 70

| 190 | 193 | 195 |
|-----|-----|-----|
| CPU | DISPLAY UNIT | INPUT UNIT |

198

| 192 | 194 | 196 | 197 | 199 |
|-----|-----|-----|-----|-----|
| ROM | RAM | STORAGE UNIT | NETWORK INTERFACE | PORTABLE STORAGE MEDIUM DRIVE |

PORTABLE STORAGE MEDIUM

191

FIG. 4B

10

90

CPU

98

| 92 | 94 | 96 | 97 | 99 |
|----|----|----|----|----|
| ROM | RAM | STORAGE UNIT | NETWORK INTERFACE | PORTABLE STORAGE MEDIUM DRIVE |

PORTABLE STORAGE MEDIUM

91

FIG. 5

FIG. 6

<SENSOR DATA DB 40>

| | UTC (ms) | GyroX (deg/sec) | GyroY (deg/sec) | GyroZ (deg/sec) | AccX (m/sec$^2$) | AccY (m/sec$^2$) | AccZ (m/sec$^2$) |
|---|---|---|---|---|---|---|---|
| Left Leg | 100000 | 61.722 | 218.763 | −32.035 | 3.585 | 7.814 | −0.235 |
| | 100010 | 38.981 | 220.753 | −40.0874 | 3.585 | 7.688 | −4.673 |
| | ... | ... | ... | ... | ... | ... | ... |
| Right Leg | 100000 | 4.2291 | 1.970 | 1.182 | 0.222 | 0.344 | 0.1111 |
| | 100010 | 4.1379 | 1.912 | 0.969 | 0.211 | 0.444 | 0.222 |
| | ... | ... | ... | ... | ... | ... | ... |
| Waist | 100000 | −81.398 | 37.383 | −1.806 | 18.899 | 111.111 | 5.555 |
| | 100010 | −85.062 | 38.019 | −4.327 | 19.222 | 111.111 | 5.555 |
| | ... | ... | ... | ... | ... | ... | ... |

EP 4 046 570 A1

FIG. 7A

FIG. 7B

[LIST OF GAIT EVENTS
(FEATURE POINTS)]

✕ HEEL CONTACT POINT

△ TOE OFF POINT

○ FASTEST SWING POINT

□ INTERMEDIATE POINT OF
DOUBLE SUPPORT SECTION

▲ POSITIVE SWING STARTING
POINT (POINT WHERE GYRO = 0)

✕ POSITIVE SWING ENDING POINT
(POINT WHERE GYRO = 0)

FIG. 7C

[LIST OF ANALYSIS TARGET
SECTIONS P]

(1)   STRIDE SECTION

(2)   SWING SECTION

(3)   DOUBLE SUPPORT SECTION

(4)   FIRST HALF OF SWING SECTION

(5)   LATTER HALF OF SWING SECTION

(6)   FIRST HALF OF DOUBLE SUPPORT SECTION

(7)   LATTER HALF OF DOUBLE SUPPORT SECTION

(8)   POSITIVE SWING SECTION

(9)   FIRST HALF OF POSITIVE SWING SECTION

(10)  LATTER HALF OF POSITIVE SWING SECTION

(11)  STANCE SECTION

FIG. 8

<ANALYSIS DATA DB 42>

| UTC | GATE INITIATION TIME | GATE FINISH TIME | GATE TIME LENGTH (s) | AVERAGE OF STRIDE TIME LENGTH (cm) | AVERAGE STEP LENGTH (cm) | AVERAGE GATE SPEED (cm/s) | NUM-BER OF STEPS | … |
|---|---|---|---|---|---|---|---|---|
| 800 | 887 | 1005 | 10.605 | 1.601 | 128.283 | 121.591 | 20 | … |
| … | … | … | … | … | … | … | … | … |

| … | FEATURE AMOUNT legGyroX_swingAll_meanNorm | FEATURE AMOUNT legGyroY_swingAll_meanNorm | … | FEATURE AMOUNT legGyroZ_swingAll_meanNorm | FEATURE AMOUNT waistGyroY_doublesupportAll_maxAbs | FEATURE AMOUNT legGyroX_doublesupportFirst_sumNorm | … |
|---|---|---|---|---|---|---|---|
| … | 80.1006 | 102.111 | … | 56.121 | 52.1006 | 63.111 | … |
| … | … | … | … | … | … | … | … |

| … | FEATURE AMOUNT waistGyroY_swingtAll_maxAbs | FEATURE AMOUNT waistGyroY_doublesupportAll_sumNorm | FEATURE AMOUNT legGyroX_swingAll_sumNorm | FEATURE AMOUNT waistGyroZ_swingAll_meanAbsDev | FEATURE AMOUNT legGyroX_doublesupportAll_sumNorm | FEATURE AMOUNT legGyroY_swingAll_meanNorm | FEATURE AMOUNT waistGyroZ_doublesupportAll_sumNorm | … |
|---|---|---|---|---|---|---|---|---|
| … | 54.982 | 43.421 | 80.1006 | 23.111 | 89.453 | 56.378 | 76.108 | … |
| … | … | … | … | … | … | … | … | … |

EP 4 046 570 A1

## FIG. 9A

### <SENSOR DATA>

| NAME | TYPE OF SENSOR DATA |
|---|---|
| waistGyroX | ANTERIOR-POSTERIOR ANGULAR VELOCITY OF WAIST |
| waistGyroY | VERTICAL ANGULAR VELOCITY OF WAIST |
| waistGyroZ | HORIZONTAL ANGULAR VELOCITY OF WAIST |
| legGyroX | ANTERIOR-POSTERIOR ANGULAR VELOCITY OF LEG |
| legGyroY | VERTICAL ANGULAR VELOCITY OF LEG |
| legGyroZ | HORIZONTAL ANGULAR VELOCITY OF LEG |
| waistAccX | ANTERIOR-POSTERIOR ACCELERATION OF WAIST |
| waistAccY | VERTICAL ACCELERATION OF WAIST |
| waistAccZ | HORIZONTAL ACCELERATION OF WAIST |
| legAccX | ANTERIOR-POSTERIOR ACCELERATION OF LEG |
| legAccY | VERTICAL ACCELERATION OF LEG |
| legAccZ | HORIZONTAL ACCELERATION OF LEG |

## FIG. 9B

### <ANALYSIS TARGET SECTION>

| NAME | ANALYSIS TARGET SECTION |
|---|---|
| swingAll | SWING SECTION |
| swingFirst | FIRST HALF OF SWING SECTION |
| swingSecond | LATTER HALF OF SWING SECTION |
| posswingAll | POSITIVE SWING SECTION |
| possingFirst | FIRST HALF OF POSITIVE SWING SECTION |
| posswingSecond | LATTER HALF OF POSITIVE SWING SECTION |
| doublesupportAll | DOUBLE SUPPORT SECTION |
| doublesupportFirst | FIRST HALF OF DOUBLE SUPPORT SECTION |
| doublesupportSecond | LATTER HALF OF DOUBLE SUPPORT SECTION |
| strideAll | STRIDE SECTION |
| stanceAll | STANCE SECTION |

FIG. 10

<STATISTICS VALUE>

| NAME | TYPE OF SENSOR SIGNAL | EQUATION |
|---|---|---|
| meanNorm | MEAN VALUE OF SUBJECT DATA | $meanNorm = \frac{1}{(N_e - Ns)} \sum_{N_s}^{N_e} X(i)$ |
| meanAbs | MEAN VALUE OF ABSOLUTE VALUES OF SUBJECT DATA | $meanAbs = \frac{1}{(N_e - Ns)} \sum_{N_s}^{N_e} |X(i)|$ |
| sumNorm | SUM OF SUBJECT DATA | $sumNorm = |\sum_{N_s}^{N_e} X(i)|$ |
| amplitude | DIFFERENCE BETWEEN MAXIMUM AND MINIMUM VALUES OF SUBJECT DATA | $amplitude = \max(X(i)) - \min(X(i))$ |
| meanDiff | MEAN VALUE OF DIFFERENTIAL VALUES OF SUBJECT DATA | $meanDiff = \frac{1}{(N_e - Ns)} \sum_{N_s}^{N_e} (X(i) - X(i-1))$ |
| sumDiff | SUM OF DIFFERENTIAL VALUES OF SUBJECT DATA | $sumDiff = \sum_{N_s}^{N_e} (X(i) - X(i-1))$ |
| stDev | STANDARD DEVIATION OF SUBJECT DATA | $stDev = \sqrt{\frac{1}{(N_e - Ns)} \sum_{N_s}^{N_e} \{X(i) - meanNorm\}^2}$ |
| meanAbsDev | DEVIATION OF ABSOLUTE VALUES OF SUBJECT DATA | $meanAbsDev = \frac{1}{(N_e - Ns)} \sum_{N_s}^{N_e} |X(i) - meanNorm|$ |
| maxAbs | MAXIMUM VALUE OF ABSOLUTE VALUES OF SUBJECT DATA | $maxAbs = \max(|X(i)|)$ |

# FIG. 11A

<PATTERN DB 44>

| ITEM NUMBER | CONTENT OF COMPENSA-TING MOVEMENT | SECTION WHERE COMPENSA-TING MOVEMENT APPEARS | FEATURE AMOUNT REPRESENTING COMPENSATING MOVEMENT | TARGET BODY PART OF COMPEN-SATED MOVEMENT | SECTION WHERE COMPEN-SATED MOVEMENT APPEARS | FEATURE AMOUNT USED AS COMPENSATED MOVEMENT | STAGE |
|---|---|---|---|---|---|---|---|
| 1 | WALK WITH PIGEON-TOED GAIT | SWING SECTION (2) | MEAN OF HORIZONTAL ROTATION ANGULAR VELOCITIES OF LEG | WAIST | DOUBLE SUPPORT SECTION (3) | LARGEST VALUE OF ABSOLUTE VALUES OF VERTICAL ROTATION ANGULAR VELOCITIES OF WAIST | COMMON |
| 2 | START TO WALK WITHOUT BENDING LEGS | FIRST HALF OF DOUBLE SUPPORT SECTION (7) | SUM OF ANTERIOR-POSTERIOR ROTATION ANGULAR VELOCITIES OF LEG | WAIST | SWING SECTION (2) | LARGEST VALUE OF ABSOLUTE VALUES OF VERTICAL ROTATION ANGULAR VELOCITIES OF WAIST | COMMON |
| 3 | WAIST MOVES UP AND DOWN | DOUBLE SUPPORT SECTION (3) | SUM OF VERTICAL ROTATION ANGULAR VELOCITIES OF WAIST | LEG | SWING SECTION (2) | SUM OF ANTERIOR-POSTERIOR ROTATION ANGULAR VELOCITIES OF LEG | SEVERE |

EP 4 046 570 A1

# FIG. 11B

<PATTERN DB 44>

| ITEM NUMBER | CONTENT OF COMPENSATING MOVEMENT | SECTION WHERE COMPENSATING MOVEMENT APPEARS | FEATURE AMOUNT REPRESENTING COMPENSATING MOVEMENT | TARGET BODY PART OF COMPENSATED MOVEMENT | SECTION WHERE COMPENSATED MOVEMENT APPEARS | FEATURE AMOUNT USED AS COMPENSATED MOVEMENT | STAGE |
|---|---|---|---|---|---|---|---|
| 4 | CIRCUM-DUCTION GAIT | SWING SECTION (2) | MEAN OF ABSOLUTE VALUES OF HORIZONTAL ROTATION ANGULAR VELOCITIES OF WAIST | LEG | DOUBLE SUPPORT SECTION (3) | SUM OF ANTERIOR-POSTERIOR ROTATION ANGULAR VELOCITIES OF LEG | SEVERE |
| 5 | WALK BY SWINGING LEGS LIKE PENDULUM | SWING SECTION (2) | MEAN OF VERTICAL ROTATION ANGULAR VELOCITIES OF LEG | WAIST | DOUBLE SUPPORT SECTION (3) | SUM OF HORIZONTAL ROTATION ANGULAR VELOCITIES OF WAIST | SEVERE |
| ... | ... | ... | ... | ... | ... | ... | ... |

EP 4 046 570 A1

FIG. 12

<SYMPTOM DB 46>

| SYMPTOM | PATTERN |
|---|---|
| MUSCULAR DYSTROPHY | 1 TO 5 |
| PARKINSON' S DISEASE | 6 TO 11 |
| … | … |

FIG. 13

```
                    ( START )
                        │
                        ▼
        ┌──────────────────────────────┐      S10
   N    │       INSTRUCTION             │
  ◄─────┤   TO START MEASURE-           │
        │      MENT IS INPUT?           │
        └──────────────────────────────┘
                        │ Y
                        ▼
        ┌──────────────────────────────┐      S12
   N    │     PREDETERMINED             │
  ◄─────┤   TIME HAS ELAPSED?           │
        └──────────────────────────────┘
                        │ Y
                        ▼
        ┌──────────────────────────────┐      S14
        │ STORE DETECTION RESULTS OF    │
        │ ANGULAR VELOCITY SENSORS AND  │
        │ ACCELERATION SENSORS IN MEMORY│
        │ TOGETHER WITH TIME INFORMATION│
        └──────────────────────────────┘
                        │
                        ▼
        ┌──────────────────────────────┐      S16
   N    │       MEASUREMENT             │
  ◄─────┤      IS FINISHED?             │
        └──────────────────────────────┘
                        │ Y
                        ▼                      S18
        ┌──────────────────────────────┐
        │ TRANSMIT SENSOR DATA (ANGULAR │
        │ VELOCITIES, ACCELERATIONS)    │
        │       TO SERVER               │
        └──────────────────────────────┘
                        │
                        ▼
                    ( END )
```

FIG. 14

```
                    ┌─────────────┐
                    │    START    │
                    └──────┬──────┘
                           │                    S30
              ┌────────────▼────────────┐
              │  STORE SENSOR DATA IN   │
              │    SENSOR DATA DB       │
              └────────────┬────────────┘
                           │                    S32
              ┌────────────▼────────────┐
              │   ACQUIRE SENSOR DATA   │
              │   FROM SENSOR DATA DB   │
              └────────────┬────────────┘
                           │                        S34
          ┌────────────────▼────────────────┐
          │  SET PATTERNS OF COMPENSATING    │
          │  MOVEMENT AND COMPENSATED MOVEMENT│
          └────────────────┬────────────────┘
                           │                        S36
          ┌────────────────▼────────────────┐
          │  EXTRACT GAIT EVENTS FROM SENSOR │
          │  DATA AND DETERMINE ANALYSIS     │
          │         TARGET SECTIONS          │
          └────────────────┬────────────────┘
                           │                      S38
            ┌──────────────▼──────────────┐
            │  CALCULATE INFORMATION ABOUT │
            │   GAIT AND FEATURE AMOUNTS   │
            └──────────────┬──────────────┘
                           │                          S40
    ┌──────────────────────▼──────────────────────────┐
    │     EXTRACT FEATURE AMOUNTS OF COMPENSATING       │
    │ MOVEMENT AND COMPENSATED MOVEMENT CORRESPONDING   │
    │   TO ONE OF PATTERNS THAT HAVE BEEN SET FROM      │
    │         AMONG CALCULATED FEATURE AMOUNTS          │
    └──────────────────────┬──────────────────────────┘
                           │                        S42
          ┌────────────────▼────────────────┐
          │ CALCULATE COMPENSATION CHANGE FROM│
          │   FEATURE AMOUNTS OF COMPENSATING │
          │   MOVEMENT AND COMPENSATED MOVEMENT│
          └────────────────┬────────────────┘
                           │
                    ◇──────▼──────◇            S44
              N    COMPENSATION
        ◇──────────CHANGES OF ALL PATTERNS THAT──────◇
                   HAVE BEEN SET HAVE BEEN
                        CALCULATED?
                           │Y
                           │                          S46
    ┌──────────────────────▼──────────────────────────┐
    │ REFER TO COMPENSATION CHANGES OF PATTERNS THAT   │
    │ HAVE BEEN SET AND DETERMINATION CONDITIONS AND   │
    │        DETERMINE STATE OF PATIENT                │
    └──────────────────────┬──────────────────────────┘
                           │                    S48
              ┌────────────▼────────────┐
              │   GENERATE SCREEN AND    │
              │  OUTPUT GENERATED SCREEN │
              └────────────┬────────────┘
                    ┌──────▼──────┐
                    │     END     │
                    └─────────────┘
```

FIG. 15

FIG. 16

GAIT STATE OF PATIENT A

<MEASUREMENT RESULT OF COMPENSATION CHANGE>

<MEASUREMENT RESULTS OF ALL BODY PARTS>

THROUGH THE MEASUREMENT OF THE GAIT STATE OF THE PATIENT A, TWO MOVEMENTS THAT ARE TYPICAL MOVEMENTS OF PARKINSON' S DISEASE SYMPTOMS ARE OBSERVED.
THE MOVEMENT APPEARING WHEN THE DISEASE IS SEVERE IS ALSO OBSERVED, AND THE SEVERITY IS EXPECTED TO BE 40%.

FIG. 17

<PATTERN DB 44>

| ITEM NUMBER | CONTENT OF COMPENSA-TING MOVEMENT | SECTION WHERE COMPENSA-TING MOVEMENT APPEARS | FEATURE AMOUNT REPRESENTING COMPENSATING MOVEMENT | TARGET BODY PART OF COMPEN-SATED MOVEMENT | SECTION WHERE COMPEN-SATED MOVEMENT APPEARS | FEATURE AMOUNT USED AS COMPENSATED MOVEMENT | STAGE |
|---|---|---|---|---|---|---|---|
| ... | ... | ... | ... | ... | ... | ... | ... |
| n | START TO WALK WITHOUT BENDING LEGS | FIRST HALF OF DOUBLE SUPPORT SECTION | SUM OF ANTERIOR-POSTERIOR ROTATION ANGULAR VELOCITIES OF LEG | AVERAGE GAIT SPEED FOR ONE MEASUREMENT | | | COMMON |
| ... | ... | ... | ... | ... | ... | ... | ... |

EP 4 046 570 A1

FIG. 18

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 21 20 2119

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 10 842 415 B1 (JAGANNATHAN RAVI [US] ET AL) 24 November 2020 (2020-11-24) <br> * figures 1-11 * <br> * column 19, line 14 - column 20, line 33 * | 1,2,4,5, 7-9,11 | INV. <br> A61B5/11 <br> A61B5/00 |
| X | US 2020/138358 A1 (CHUNG PAU-CHOO [TW] ET AL) 7 May 2020 (2020-05-07) <br> * figure 1 * <br> * paragraph [0017] - paragraph [0039] * | 1-9,11 | |
| X | WO 2017/065241 A1 (TOKYO INST TECH [JP]) 20 April 2017 (2017-04-20) <br> * figures 1, 3 * <br> * the whole document * | 1-9,11 | |
| X | JP 2011 177278 A (TOKYO INST TECH) 15 September 2011 (2011-09-15) <br> * paragraph [0020] - paragraph [0057] * <br> * figures 1-11 * | 1-9,11 | |

**TECHNICAL FIELDS SEARCHED (IPC)**

A61B

## INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| The Hague | 15 July 2022 | Knoop, Jan |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04E07)

Europäisches
Patentamt

European
Patent Office

Office européen
des brevets

INCOMPLETE SEARCH
SHEET C

Claim(s) completely searchable:
        1-9, 11

Claim(s) not searched:
        10

Reason for the limitation of the search (non-patentable invention(s)):

1.1        The method of claim 10 is not patentable since it is a diagnostic method within the meaning of Article 53(c) EPC.
1.1.1       According to the decision of the Enlarged Board of Appeal G 1/04 (see also the Guidelines G-II, 4.2 and 4.2.1.3) a method qualifies as a diagnostic method in the sense of Article 53(c) if the method comprises the following steps (G 1/04, point 5 of the reasons):
1.2        (a) the examination phase involving the collection of data,
1.3        (b) the comparison of these data with standard values,
1.4        (c) the finding of any significant deviation, i.e. a symptom, during the comparison, and
1.5        (d) the attribution of the deviation to a particular clinical picture, i.e. the deductive medical or veterinary decision phase,
1.6        wherein the steps of a technical nature belonging to steps (a) to (c) must satisfy the criterion "practised on the human or animal body" (see conclusion, point 3).
1.7        The criterion "practised on the human or animal body" is to be considered only in respect of method steps which are of a technical nature (see points 6.4.1 and 6.4.4 of G 1/04). Thus, it neither applies to the deductive decision phase, nor to the above-mentioned steps b) and c) which consist in comparing the data collected in the examination phase with standard values and in finding a significant deviation resulting from the comparison. These activities are predominantly of a non-technical nature and normally not practised on the human or animal body (see point 6.4.1 of G 1/04).
1.7.1      Present claim 10 relates to a gait state determination method.
2        Step (a) mentioned as above is disclosed in claim 10 by the step of "acquiring data of a movement of at least a part of a body of a person ... using a sensor attached to the body of the person". This step is of technical character since it inherently involves technical means. Further, this step obviously requires the presence of the human body, since no information can be obtained otherwise.
2.1        Step (b) which refers to the comparison of the data collected with standard values is to be found in the following feature: "identifying a first feature amount of a first movement from the data acquired ... the first movement being determined in advance and corresponding to a symptom of the person". Here, it should be underlined that a symptom is a disease, see e. g. p. 5, l. 10-11.
2.2        Step (c) which refers to the finding of any significant deviation, i.e. a symptom, during the comparison and step (d) which refers to the attribution of the deviation to a particular clinical picture, i.e. the diagnosis stricto sensu, is to be found in the step of "making a determination regarding the gait state", wherein the application discloses (see par. 0043): "the gait state of the patient, i.e., the presence or absence of the symptom and the severity based on the calculated compensation change."

32

Europäisches
Patentamt

European
Patent Office

Office européen
des brevets

**INCOMPLETE SEARCH
SHEET C**

2.2.1      Consequently, the method according to claim 10 includes all the features of a diagnostic method practised on the human or animal body as defined in the decision G 1/04. Such a method is exempted from patentability by Article 53(c) EPC.

## ANNEX TO THE EUROPEAN SEARCH REPORT
## ON EUROPEAN PATENT APPLICATION NO.

EP 21 20 2119

This annex lists the patent family members relating to the patent documents cited in the above-mentioned European search report.
The members are as contained in the European Patent Office EDP file on
The European Patent Office is in no way liable for these particulars which are merely given for the purpose of information.

15-07-2022

| Patent document cited in search report | | Publication date | Patent family member(s) | | Publication date |
|---|---|---|---|---|---|
| US 10842415 | B1 | 24-11-2020 | US | 10842415 B1 | 24-11-2020 |
| | | | WO | 2021080967 A1 | 29-04-2021 |
| US 2020138358 | A1 | 07-05-2020 | NONE | | |
| WO 2017065241 | A1 | 20-04-2017 | JP | 6951750 B2 | 20-10-2021 |
| | | | JP | 2021184964 A | 09-12-2021 |
| | | | JP | WO2017065241 A1 | 09-08-2018 |
| | | | WO | 2017065241 A1 | 20-04-2017 |
| JP 2011177278 | A | 15-09-2011 | JP | 5607952 B2 | 15-10-2014 |
| | | | JP | 2011177278 A | 15-09-2011 |

EPO FORM P0459

For more details about this annex : see Official Journal of the European Patent Office, No. 12/82

**REFERENCES CITED IN THE DESCRIPTION**

**Patent documents cited in the description**

- JP 2012179114 A